# EUROPEAN PATENT APPLICATION

(11) **EP 1 260 626 A2**
(43) Date of publication of application: **27.11.2002**
(21) Application number: 02018122.8
(22) Date of filing: 28.01.1997
(51) Int. Cl.: D04H 1/64, A61L 15/60, A61F 13/15

(54) **Process for bonding fibrous web**

(62) Divisional of application: 97905004.4
(71) Applicant: CAMELOT SUPERABSORBENTS LTD, Calgary, Alberta T2E 7L6 (CA)
(72) Inventor: Bouman, Dirk, 7521 AZ Enschede (NL); Shaltry, Michael J., Charlotte, NC 28120 (US)
(74) Representative: Ritter, Stephen David

(57) **Abstract**

A process for forming a fibrous web in the presence of a water-absorbent material, such as a superabsorbent, in which a web of non-water-absorbent fibers and water-absorbent materials is formed and then contacted with an aqueous solution of a binder such as latex in an amount of 5 wt.%, or an uncured binder.

## Description

The present invention relates to a process for bonding a fibrous web which includes water-absorbent material such as water-absorbent fibers. In particular the present invention relates to bonding a fibrous web containing so-called superabsorbent fibers. The invention also relates to an article of manufacture comprising a fibrous web made in accordance with the process.

Fibrous webs containing water-absorbent compositions are widely used in the manufacture of products which require high absorbtion capability; for example, surgical and dental sponges, tampons, sanitary napkins and pads, bandages, disposable diapers, meat trays, filters, spill control materials, waste management materials, cable wrap, protective articles such as operating gowns and household pet litter. Fibrous webs containing water-absorbent compositions are also used in the modification of soil to improve water-retention and increase air capacity and in a host of other applications.

Water-absorbent compositions suitable for use in these fibrous webs may be in any suitable form including powders, fibers and filaments, with fibers being particularly preferred.

As used herein, the term "water" when used in the phrases "water-absorbing", "water-absorbent" and "water-swellable" is understood to mean not only water but also aqueous media such as electrolyte solutions, such as body fluids.

The fibrous webs of the type with which the present invention is concerned are generally formed from non-water-absorbent fibers. By "non-water-absorbent" we mean that the fibers do not absorb water to an appreciable extent. Suitable materials from which these fibers may be formed include natural materials such as wood pulp or cellulose or synthetic materials such as synthetic cellulose, viscose, polyester, non-water-absorbent polymers of ethylene, non-water-absorbent polymers of propylene, polyamide and ethylene-propylene copolymer fibers, and mixtures thereof with polyester, polyethylene and polypropylene fibers being particularly preferred. Particularly preferred fibers are those selected from the group consisting of rayon fibers, cellulose ester fibers, protein fibers, polyamide fibers, polyester fibers, polyvinyl fibers, polyolefin fibers, polyurethane fibers, aramid fibers, glass fibers and mixtures thereof. In one alternative, the non-water-absorbent fibers may be, or may fibers having hollow cores such as the polyester, typically polyethylene terephthalate fibers commercially available from E.I. DuPont de Nemours under the trade mark HOLLOWFILL.

The fibrous web additionally includes water-absorbent compositions, which are preferably water-absorbent fibers.

A number of water-absorbent compositions have been developed which exhibit the capacity to be water-absorbing. For example, U.S. Patent numbers 3,954,721 and 3,983,095 disclose preparations for derivatives of copolymers of maleic anhydride with at least one vinyl monomer in fibrous form. The fibrous copolymers are rendered hydrophillic and water-swellable by reaction with ammonia or an alkali metal hydroxide. U.S. Patent No. 3,810,468 discloses lightly cross-linked olefin-maleic anhydride copolymers prepared as substantially linear copolymers and then reacted with a diol or a diamine to introduce cross-linking. The resultant lightly cross-linked copolymers are treated with ammonia or an aqueous or alcohol solution of an alkali metal hydroxide. U.S. Patent No. 3,980,663 describes water-swellable absorbent articles made from carboxylic polyelectrolytes via cross-linking with glycerine diglycidyl ether. These patents are incorporated herein by reference.

European Published Application No. 0 268 498 (incorporated herein by reference) describes a water-absorbent composition formed by causing a substantially linear polymer of water-soluble ethylenically unsaturated monomer blends comprising carboxylic and hydroxylic monomers to cross-link internally.

Further examples of water-absorbent compositions are those produced from a copolymer of an α,β unsaturated monomer having at least one pendant unit selected from a carboxylic acid group and derivatives thereof and a copolymerisable monomer. A proportion of the pendant units are present in the final copolymer as the free acid and a proportion as the salt of the acid. These copolymers are capable of being cross-linked, either internally or with a variety of cross-linking agents, to form the water-swellable composition. Examples of water-swellable compositions of this type can be found in U.S. Patent Nos 4,616,063, 4,705,773, 4,731,067, 4,743,244, 4,788,237, 4,813,945, 4,880,868 and 4,892533 and European Patent Nos 0 272 074 and 0 264 208 and European Published Application No. 0 436 514 which are incorporated herein by reference.

Derivatives of carboxylic acid groups include carboxylic acid salt groups, carboxylic acid amide groups, carboxylic acid imide groups, carboxylic acid anhydride groups and carboxylic acid ester groups.

Other examples of water-absorbent compositions can be found in European Published Application Nos 0 269 393, 0 342 919 and 0 397 410, British Patent Application Nos 2 082 614 and 2 126 591, U.S. Patent Nos 4,418,163, 4,418,163 3,989,586, 4,332,917, 4,338,417, 4,420,588 and 4,155,957 and French Patent Application No. 2 525 121 which are incorporated herein by reference.

The fibrous web may be formed as a non-woven web by any conventional means. Suitable means include air-laying, wet-laying and carding. The fibers are then bonded, for example by the action of an adhesive and/or heat. However, aqueous solutions of adhesives have not generally been utilised when the web contains water-absorbent compositions as it is believed that the water in the aqueous solution will be absorbed by the water-absorbent compositions which, having been activated, will not function satisfactorily in use. Additionally or alternatively, the aqueous solution of the adhesive may coat the water-absorbent composition, rendering it ineffective.

In EP 0 357 474 an aqueous adhesive solution is used to bond the fibers in the web. However, such bonding takes place before the water-absorbent material is introduced into the web. In particular, an air-laid web of non-water-absorbent fibers is first sprayed on one side with a latex binder before being passed through an oven where the latex binder is at least partially cured. The web is then sprayed with precursors of the water-absorbing composition onto the side of the web remote from that on which the latex was sprayed. The web is then passed through a dryer where cross-linking occurs to form the superabsorbent *in situ.*

In US 5128082 and US 5378528 the aqueous binder is applied to a fibrous web which incorporates a superabsorbent material. As in EP 0357474 the binder, which is preferably latex, is cured to bind the fibrous web. The amount of latex used is in the range of 5 to 30 weight percent of the final structure, and preferably from about 10 to 20 weight percent.

The use of an aqueous solution of an adhesive such as latex is also described in WO 90/11184. Here, an aqueous solution of a binder is used to bind water-absorbent particles to the fibers of the web. The particles of the superabsorbent are preferably added after the fibers have been wetted by the binder such that the binder does not coat the particles and thereby interfere with their absorbency.

The known processes which involve the use of aqueous binders have certain disadvantageous and drawbacks. In particular, the binder, whether applied before or after the inclusion of the superabsorbent composition into the web, reduces the absorbency of the resultant web. In addition, the process in which the binder is applied before the web is sprayed with the precursors of the water-absorbing polymer is unsuitable where it is desired that the superabsorbent is to be in the form of fibers.

We have now discovered the aforemention problems may be reduced or obviated if the binder used to bind the fibers of the fibrous web is used in an amount of less than 5 weight percent of the structure being formed. This is particularly suitable where the web includes the water-absorbent material in fibrous form. Surprisingly, this reduced amount of binder is sufficient to bind the web. One benefit of using this reduced amount of binder is that the absorbency of the web formed is higher than has been previously achieved using aqueous binders in accordance with known techniques.

Thus, according to a first aspect of the present invention there is provided a process for bonding a fibrous web in the presence of water-absorbent material wherein the pre-formed web is contacted with an aqueous solution of a binder, the binder being used in an amount of less than 5 weight percent of the fibrous web.

Suitable binders include cationic starch, polyvinyl alcohol, pearl starch, natural gums, such as tragacanth, karaya and quar, vinyl acetate-acrylic acid copolymers, polyvinyl chlorides, polyvinyl acetates, ethylene-vinyl acetates, styrenebutadiene carboxylates, natural latex, synthetic latex, including polyacrylates such as polyethylacrylate and copolymers thereof, polyacrylonitriles and thermosetting cationic resins such as urea formaldehyde resins and polyamide-epichlorhydrin resins, with natural or synthetic latex being preferred. Examples of commercially available compositions which are suitable binders include AIRBOND, AIRFLEX and VINAC of Air Products Inc., HYCAR and GEON of Goodrich Chemical Co., and FULATEX of HB Fuller Company.

The binder may be applied to the web by any suitable means. In one arrangement, the web may be immersed in the binder; excess binder can then be removed by, for example, applying pressure to the web. In one alternative arrangement, the binder is sprayed onto the web. The binder may be sprayed onto one or both sides of the web. Spray bonding is the generally preferred means of applying the binder to the web. It is particularly preferred in applications where the retention of the bulk or loft of the web is important. The binder may be sprayed onto the fibrous web to obtain spot welds at the points where fibers are in mutual contact. The binder is preferably used in an amount of from 1wt % to 5wt %, more preferably 2wt % to 4wt %. Amounts of less than 1wt % may also be used.

The treated web is then allowed to cure. Curing is preferably achieved by passing the web that has been treated with the aqueous solution of the binder through an oven. During curing, cross-linking of the binder will occur and binding will thereby be effected. In order to effect cross-linking the binder will contain an amount of a suitable cross-linking agent. One suitable cross-linking agent is N-methylol acrylamide.

The binder may include suitable additives such as defoamers, surfactants, external crosslinkers, thickeners, flame retardants, catalysts, pH adjusters, dyes and pigments, fillers, optical brighteners, sewing aids and water repellents.

We have also discovered an improved process for forming a fibrous web in the presence of water-absorbent material wherein the binder is allowed to dry but does not cure to an appreciable extent. This process also overcomes many of the aforementioned problems.

Thus, according to the second aspect of the present invention there is provided a process for forming a fibrous web in the presence of water-absorbent material wherein the binder being free of cross-linker is applied to the fibrous web and allowed to dry.

Any suitable binder may be used provided that it is capable of being provided in aqueous form and of hardening on removal of the water. The binders listed in connection with the above-first aspect of the invention are particularly suitable provided that they do not include a cross-linking agent.

A preferred binder is an aqueous solution of the uncured polymer from which water-absorbent compositions may be formed. A particularly suitable binder is an aqueous solution of the uncured polymer from which the water-absorbent composition present in the web is formed.

We have found that a particularly suitable binder is a composition comprising a copolymer having pendant carboxylic acid groups or other groups convertible to carboxylic acid groups, wherein at least a portion of the carboxylic acid groups are partially neutralised. The copolymer may be formed from an α,β-unsaturated monomer having at least one pendant unit selected from a carboxylic acid group and derivatives thereof and a copolymerisable monomer.

The use of the uncured polymer from which the water-absorbent composition is formed as a binder results in a web having improved absorbency. Further, as the polymer of the binder is one from which the water-absorbent composition may be formed, the binder will be compatible with the water-absorbent composition and will not have a deleterious effect thereon.

The binder formed from the polymer from which a water-absorbent composition may be formed may undergo some crosslinking on drying and fall within the definition of the second aspect of the present invention. If cross-linking occurs, enhanced absorbency of the web will be achieved. Crosslinking may be internal cross-linking or, alternatively, the binder solution may contain some external cross-linking agents. Suitable cross-linking agents include: monomers containing at least two hydroxyl groups such as alkylene glycols containing 2-10 carbon atoms and their ethers, cycloalkylene glycols, Bisphenol A, hydroxy alkylene derivatives of Bisphenol A, hydroquinone, phloroglucinol, hydroxy alkylene derivatives of diphenols, glycerol, erythritol, pentaerythritol, and mono-, di, or oligo-saccharides; heterocyclic carbonates; and monomers containing at least one amine group and at least one hydroxyl group such as ethanolamine, tris (hydroxymethyl) aminomethane, 3-amino-1-propanol, DL-1-amino-2-propanol, 2-amino-1-butanol, N,N-dimethylethanolamine, diisopropanol-amine methyl diethanol amine, triethanol amine, 2-(methylamino)ethanol and the like.

Particularly suitable uncured, in the sense of the invention, copolymers for use as a binder in the present invention contain from about 25 to about 75 mole percent recurring units of the α,β-unsaturated monomer and from about 75 to about 25 mole percent recurring units of a copolymerisable monomer. The copolymer preferably contains from about 35 to about 65 mole percent of recurring units of at least one α,β-unsaturated monomer and from about 65 to about 35 mole percent of at least one copolymerisable co-monomer. Most preferably, the copolymer will be an equimolar copolymer.

Suitable α,β-unsaturated monomers are those bearing at least one pendant carboxylic acid unit or derivative of a carboxylic acid unit. Derivatives of carboxylic acid units include carboxylic acid salt groups, carboxylic acid amide groups, carboxylic acid imide groups, carboxylic acid anhydride groups and carboxylic acid ester groups.

Examples of suitable α,β-unsaturated monomers include maleic acid, crotonic acid, fumaric acid, mesaconic acid, the sodium salt of maleic acid, the sodium salt of 2-methyl, 2-butene dicarboxylic acid, the sodium salt of itaconic acid, maleamic acid, maleamide, N-phenyl maleimide, maleimide, maleic anhydride, fumaric anhydride, itaconic anhydride, citraconic anhydride, ethyl maleic anhydride, diethylmaleate, methylmaleate and the like and mixtures thereof.

Any suitable copolymerisable co-monomer can be employed. Examples of suitable co-monomers include ethylene, propylene, isobutylene, C₁ to C₄ alkyl methacrylates, vinyl acetates, methyl vinyl ether, isobutyl vinyl ether and styrenic compounds having the formula: 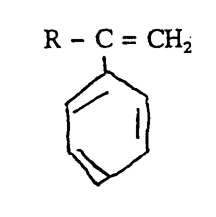 wherein R represents hydrogen or an alkyl group having from 1 to 6 carbon atoms, and wherein the benzene ring is substituted or unsubstituted.

Suitable alkyl acrylates include methyl acrylate, ethyl acrylate, isopropyl acrylate, n-propyl acrylate, n-butyl acrylate, and the like and mixtures thereof.

Suitable alkyl methacrylates include methyl methacrylate, ethyl methacrylate, isopropyl methacrylate, n-propylmethacrylate, n-butyl methacrylate, and the like and mixtures thereof.

One alternative uncured polymer is formed from a water-soluble blend of monoethylenically unsaturated monomers. the polymer may include a comonomer. The polymer preferably comprises 50 to 95% by weight of an ethylenically unsaturated monomer and 5 to 50% by weight of a copolymerisable ethylenically unsaturated monomer.

Preferred carboxylic monomers include methacrylic acid, acrylic acid, maleic acid or anhydride, itaconic acid and ethylenically unsaturated carboxylic acids or anhydrides. Some of the carboxylic monomer units in the copolymer may be replaced by monomer units derived from an ethylenically unsaturated sulphonic acid such as 2-acrylamido-2-methylpropane sulphonic acid or allyl sulphonic acid.

The or each copolymerisable ethylenically unsaturated monomer may be selected from a water-soluble ethylenically unsaturated monomer such as acrylamide or a water-insoluble monomer, for example an olefin, such as isobutylene, an aromatic ethylenically unsaturated monomer, such as styrene or a substituted styrene, an alkyl ester of acrylic or methacrylic acid, such as methyl or ethyl acrylate or methacrylate, butyl acrylate or methacrylate or 2-ethylhexyl acrylate or methacrylate, vinyl acetate or acrylonitrile. Other monomers that may be used include ethylenically unsaturated monomers that carry a pendent group of the formula -AₘBₙAₚR where B is ethyleneoxy, n is a integer of at least 2, A is propyleneoxy or butyleneoxy, m and p are each an integer less than n and preferably below 2 and most preferably zero, and R is a hydrophobic group containing at least 8 carbon atoms as described in more detail in EP-A-213799. The comonomers(s) are generally present in amounts of at least 5% and preferably at least 10% by weight based on the monomers used for forming the copolymer, and they may be present in amounts up to about 50%, generally below 45%, by weight.

The fibrous web produced in accordance with the above-mentioned first or second aspect of the invention is suitable for use in the production of articles of manufacture.

Thus, in accordance with a third aspect of the prcsent invention, there is provided an article of manufacture comprising a fibrous web made in accordance with the process of the above first or second aspect.

The article of manufacture is preferably selected from the group consisting of: disposable diapers; sanitary napkins; tampons; pant liners; adult incontinence pads; coverstock for feminine hygiene products; surgical and dental sponges; bandages; patient underpads; wipes; domestic wipes; industrial wipes; packaging; medical tray pads; fenestration drapes; filters; spill control materials; waste management materials; protecive articles; operating gowns; mortuary pads; cable wrap; food tray pads; food preservation articles; seed germination pads; household pet litter; roofing materials; automotive trim; furniture; bedding; clothing; and soil modifiers.

Where the article of manufacture comprises a fibrous web made in accordance with the process of the above second aspect of the invention, the binder is sufficient to retain the integrity of the web during use but is sufficiently degradable in the presence of a large volume of water, particularly turbulent water such as is found in a toilet, that the article is particularly suitable for disposal in water, such as in a toilet.

Preferred embodiments of the present invention will now be described with reference to the accompanying drawing in which:

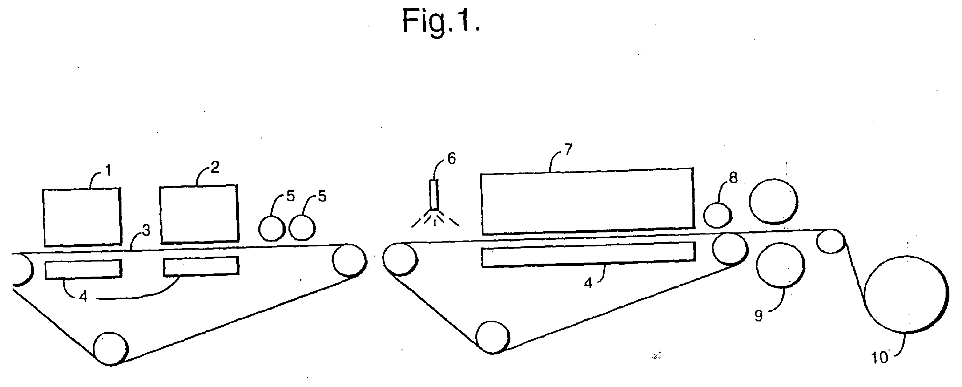

- **Figure 1**: is a schematic flow diagram outlining the process of the present invention.

In the following examples, the non-water-absorbent fibers and the superabsorbent fibers are fed to formers 1 and 2. In the pilot plant, a Laroche 400 fiber feed was employed to feed fibers to the formers. The fibers are fed from the formers onto a belt 3 comprising the forming table. The fibers are held in place by the action of vacuum boxes 4. Two light compactor rolls 5 are used to densify and compact the web.

The aqueous solution of the binder (latex) optionally containing a cross-linking agent was sprayed onto one side of the web. The spray system 6 is located between the end of the forming table 3 and the dryer 7. In an alternative arrangement a second spray system and dryer (not shown) may be located after the dryer 7. This second spray system may spray the binder onto the side of the web opposite to that sprayed on the first side.

The binder may contain a cross-linking agent or may be free of cross-linker. As the web passes through the dryer 7. The dryer, which is preferably an air dryer, causes the water to evaporate. Where the binder includes a cross-linker, the binder will cure and thereby strengthens. Where the binder is free of cross-linker, the binder dries as it passes through the dryer and thereby strengthens. In addition, where the non-water-absorbent fibers of the web are thermal bonding fibers, the dryer 7 activates these fibers. A compactor roll 8 may be located within the dryer.

Calendaring rolls 9 may be located after the dryer. These rolls may be used to emboss the web, reduce calliper or modify the surface of the web.

The final bound web preferably has a density of from 0.05 to 0.2 gcm⁻¹

### Examples 1-6

Using the above-described process, fibrous webs of the compositions set out in Table 1 were provided.

**Table 1**

| Run No | Fluff (wt%) | Superabsorbent Fiber (wt%) | Latex (wt%) | Basis weight (g/m²) |
|---|---|---|---|---|
| 1 | 71 | 25 | 4 | 124 |
| 2 | 76 | 20 | 4 | 102 |
| 3 | 76 | 20 | 4 | 155 |
| 4 | 60 | 36 | 4 | 154 |
| 5 | 66 | 30 | 4 | 131 |
| 6 | 100 | 0 | 4 | 120 |

By 'fluff', we mean all components of the web other than the superabsorbents and latex.

The absorbency of the webs produced in Examples 1 to 6 were tested in accordance with the following test procedure:
A 10 x 30 cm strip of the sample was cut from the absorbent material to be tested. Approximately 2 cm of the specimen was immersed into 0.9% saline and the weight increase due to absorption was measured after 30 minutes. The results of the tests are set out in Table 2

**Table 2**

| Run No | Absorption (g) |
|---|---|
| 1 | 24 |
| 2 | 23 |
| 3 | 36 |
| 4 | 44 |
| 5 | 27 |
| 6 | 16 |

### Comparative Test

Thus it will be understood that even though an aqueous solution of a binder is used to bind the web, the water-absorbent composition functions to enhance the absorbency of the web.

## Claims

1. A process for forming a non-woven fibrous web containing superabsorbent material using a binder wherein the binder is substantially free of cross-linker and in said process binder is applied to the fibrous web and allowed to dry but does not cure to an appreciable extent.

2. A process according to Claim 1 wherein the binder is selected from the group consisting of cationic starch, polyvinyl alcohol, pearl starch, natural gums such as tragacanth, karaya and guar, vinyl acetate-acrylic acid copolymers, polyvinyl chlorides, polyvinyl acetates, ethylene-vinyl acetates, styrenebutadiene carboxylates, natural latex, synthetic latex, including polyacrylates such as polyethylacrylate and copolymers thereof, polyacrylonitriles and thermosetting cationic resins such as urea formaldehyde resins and polyamide-epichlorhydrin resins.

3. A process according to Claim 1 wherein the binder is natural or synthetic latex.

4. A process according to Claim 1 wherein the binder is an aqueous solution of a polymer from which a superabsorbent composition may be formed by curing.

5. A process according to any one of Claims 1 to 4 wherein the binder is applied to the fibrous web by spraying.

6. An article of manufacture comprising a fibrous web obtainable by the process of any one of Claims 1 to 5.

7. An article of manufacture according to Claim 6 selected from the group consisting of: disposable diapers; sanitary napkins; tampons; pant liners; adult incontinence pads; coverstock for feminine hygiene products; surgical and dental sponges; bandages; patient underpads; wipes; domestic wipes; industrial wipes; filters; spill control materials; waste management materials; protective articles; operating gowns; packaging; medical tray pads; fenestration drapes; mortuary pads; cable wrap; food tray pads; food preservation articles; seed germination pads; household pet litter; roofing materials; automotive trim; furniture; bedding; clothing; and soil modifiers.

8. An article of manufacture according to Claim 6 or Claim 7 wherein said binder is an aqueous solution of an uncured polymer comprising from about 25 to about 75 mole percent recurring units of at least one α,β-unsaturated monomer bearing at least one pendant unit selected from the group consisting of carboxylic acid units and derivatives of carboxylic acid units, and from about 75 to about 25 mole percent recurring units of at least one monomer copolymerisable with said at least one α,β-unsaturated monomer.
